# EUROPEAN PATENT APPLICATION

(11) **EP 4 621 391 A1**
(43) Date of publication of application: **24.09.2025**
(21) Application number: 25160516.8
(22) Date of filing: 27.02.2025
(51) Int. Cl.: G01N 21/78, G01N 21/84

(54) **SYSTEM, METHOD, AND DEVICE FOR DETERMINING CONCENTRATION OF A TARGET GAS**

(30) Priority: 19.03.2024 CN 202410315773
(71) Applicant: Life Safety Distribution GmbH, 1180 Rolle (CH)
(72) Inventor: HE, Shawn, Charlotte, 28202 (US); YU, Yan, Charlotte, 28202 (US); HUANG, Chuang, Charlotte, 28202 (US); TANG, Huan, Charlotte, 28202 (US)
(74) Representative: Haseltine Lake Kempner LLP

(57) **Abstract**

A system for determining concentration of a target gas is disclosed. The system comprises a sensing chamber comprising at least one sampling bag configured to receive the target gas. Further, a testing paper module having at least one testing paper that is configured to change color based at least on concentration of the target gas. A detecting device operationally coupled to the sensing chamber, comprising a housing having at least one testing chamber configured to receive the testing paper module. Further, at least one controller is configured to facilitate flow of the target gas through the testing paper module by actuating at least one pump. The flow of the target gas facilitates to change color of the at least one testing paper. Further, the concentration of the target gas is determined via at least one detector, based on the change in color of the at least one testing paper.

## Description

### FIELD OF THE DISCLOSURE

The present invention relates to a concentration detection of a target gas. More particularly, the invention relates to a method and a system to determine concentration of respiratory gasses.

### BACKGROUND OF THE DISCLOSURE

Many respiratory gasses are found in atmosphere and also produced in a human body. The respiratory gasses such as Oxygen, Nitric oxide and carbon dioxide regulates blood pressure, transmits signals between neurons and suppresses pathogens. Excessive production of these gasses leads to many problems. For example, excess amount of NO gas may be vulnerable for the human body. High concentration of NO gas in the human body may damage host cells causing neurotoxicity during strokes and also may cause hypotension. On the other side, low levels of NO gas may affect blood circulation causing high blood pressure, poor vision, fatigue, memory loss etc. Therefore, regular monitoring of these gasses is an essential practice to ensure ideal function of the human body. Many devices and systems are widely used in the market for monitoring the concentration of respiratory gasses. However, the available solutions do not allow easy sample collection of these gasses from a user, they do not provide accurate concentration levels and require external interferences for carrying concentration detection of the respiratory gasses.

Applicant has identified various issues and challenges associated with the current solutions for measuring concentration of NO gas. However, with creativity, hard work, and innovation, the present disclosure has addressed many of these problems through its methods and equipment.

### SUMMARY OF THE DISCLOSURE

The following presents a simplified summary in order to provide a basic understanding of some aspects of the present disclosure. This summary is not an extensive overview and is intended to neither identify key or critical elements nor delineate the scope of such elements. Its purpose is to present some concepts of the described features in a simplified form as a prelude to the more detailed description that is presented later.

In an example embodiment, a detecting device is disclosed. The detecting device comprises a housing having at least one testing chamber configured to receive a testing paper module having at least one testing paper. Further, a pressing block inside the housing, is configured to channelize flow of target gas from the testing paper module to the pressing block. The flow of the target gas facilitates to penetrate the target gas through the at least testing paper and change color of the at least one testing paper. Further, at least one detector is configured to monitor the change in color of the at least one testing paper. Further, at least one controller is communicatively coupled to the at least one detector and configured to determine concentration of the target gas based at least on the change in color of the at least one testing paper.

In some embodiments, the housing is fabricated with at least one slot to insert the testing paper module inside the at least one testing chamber. Further, the slot further comprises at least one locking unit configured to fixedly couple the testing paper module with the at least one slot.

In some embodiments, the housing further comprises at least one pump fluidly coupled to the pressing block via a second tube. Further, at least one pushing mechanism coupled to the pressing block via a pushing block. Further, the at least one pushing mechanism actuates upon receiving a command from the at least one controller, that in turn linearly extends the pushing block to vertically actuate the pressing block and position over the at least one testing paper, upon activating the detecting device. Further, the at least one pump actuates to facilitate flow of the target gas to facilitate change in color of the at least one testing paper, upon positioning the pressing block over the at least one testing paper.

In some embodiments, the housing further comprises at least one illuminating device positioned in proximity to the at least one detector and coupled to the at least one controller. The at least one illuminating device is configured to illuminate the at least one testing paper to facilitate the at least one detector to monitor the change in color of the at least one testing paper. Further, the at least one illuminating device comprises at least one of light emitting diode (LED) light.

In some embodiments, the housing is enclosed with a cover, the cover comprises a power button configured to facilitate electrical power to the detecting device. Further, at least one result screen is configured to display the concentration of the target gas to a user.

In some embodiments, the pressing block further comprises a channel configured to facilitate flow of the target gas within the pressing block. Further, a sealing gasket configured to seal the channel to prevent leakage of the target gas flowing between the testing paper module and the pressing block. The pressing block is further coupled to a spring that is configured to retract the pressing block at an initial position upon determining the concentration of the target gas.

In another example embodiment, a method is disclosed. The method comprises, creating, via a pressing block, a channel between the sensing chamber and a detecting device to facilitate the flow of target gas. Further, facilitating, via at least one pump, the flow of the target gas from the channel between the sensing chamber and the detecting device. Further, monitoring, via at least one detector, change in color of the at least one testing paper. Further, determining, via at least one controller, concentration of the target gas based at least on the change in color of the at least one testing paper.

In some example embodiments, the method further comprises actuating, via at least one pushing mechanism, the pressing block to facilitate translation movement of a pushing block. Further, pressing, via the pushing block, the pressing block to align the pressing block over the at least one testing paper to facilitate flow of the target gas via the at least one testing paper.

In another example embodiment, a system is disclosed. The system comprises a sensing chamber comprising at least one sampling bag configured to receive the target gas. Further, a testing paper module having at least one testing paper that is configured to change color based at least on concentration of the target gas. Further the system comprises, a detecting device operationally coupled to the sensing chamber, comprising a housing having at least one testing chamber configured to receive the testing paper module. Further, at least one controller configured to facilitate flow of the target gas through the testing paper module by actuating at least one pump. The flow of the target gas facilitates to change color of the at least one testing paper. Further, determine concentration of the target gas via at least one detector, based at least on the change in color of the at least one testing paper.

In some embodiments, the testing paper module further comprises a gas channel that is configured to channelize the target gas from the at least one sampling bag to the at least one testing paper. Further, the gas channel is fluidly coupled to the at least one sampling bag via a first tube to facilitate flow of the target gas from the at least one sampling bag to the at least one testing paper.

In some embodiments, the housing further comprises at least one pushing mechanism coupled to a pressing block via a pushing block. Further, at least one illuminating device positioned in proximity to the at least one detector, configured to illuminate the at least one testing paper to facilitate the at least one detector to monitor the change in color of the at least one testing paper.

In some embodiments, the at least one illuminating device comprises at least one of light emitting diode (LED) light.

In some embodiments, the housing is enclosed with a cover. The cover comprises a power button configured to facilitate electrical power to the detecting device. Further, an on/off button is configured to activate or deactivate the detecting device. Further, at least one result screen is configured to display the concentration of the target gas to a user.

In some embodiments, the pressing block comprises a channel configured to facilitate flow of the target gas from the pressing block to the at least one pump. Further, a sealing gasket configured to seal the channel to prevent leakage of the target gas flowing between the testing paper module and the pressing block.

In some embodiments, the at least one controller is electrically coupled to at least one result screen configured to display the determined concentration of the target gas to a user.

The above summary is provided merely for purposes of summarizing some example embodiments to provide a basic understanding of some aspects of the invention. Accordingly, it will be appreciated that the above-described embodiments are merely examples and should not be construed to narrow the scope or spirit of the invention in any way. It will be appreciated that the scope of the invention encompasses many potential embodiments in addition to those here summarized, some of which will be further described below.

### BRIEF DESCRIPTION OF THE DRAWINGS

Having thus described certain example embodiments of the present disclosure in general terms, reference will hereinafter be made to the accompanying drawings, which are not necessarily drawn to scale, and wherein:
FIG. 1A is a perspective view of a sensing chamber, in accordance with an example embodiment of the present disclosure;
FIG. 1B is an exploded view of a testing paper module of the sensing chamber, in accordance with the example embodiment of the present disclosure;
FIG. 2A is a perspective view of a detecting device, in accordance with the example embodiment of the present disclosure;
FIG. 2B illustrates an internal view of the detecting device, in accordance with the example embodiment of the present disclosure;
FIG. 3A illustrates coupling of the testing paper module with the detecting device, in accordance with the example embodiment of the present disclosure;
FIG. 3B illustrates interlocking of a gas channel within a slot, in accordance with the example embodiment of the present disclosure;
FIG. 4A illustrates horizontal movement of a pushing block with respect to a pressing block, in accordance with the example embodiment of the present disclosure;
FIG. 4B illustrates vertical movement of the pressing block by the pushing block, in accordance with the example embodiment of the present disclosure;
FIG. 5A illustrates position of the pressing block with respect to the at least one testing paper before pushing of the pressing block, in accordance with the example embodiment of the present disclosure;
FIG. 5B illustrates position of the pressing block with respect to the at least one testing paper after pushing of the pressing block, in accordance with the example embodiment of the present disclosure;
FIG. 5C illustrates flow of the target gas from the sensing chamber to the detecting device, in accordance with the example embodiment of the present disclosure;
FIG. 6 illustrates monitoring of change in color of the at least one testing paper by the at least one detector, in accordance with the example embodiment of the present disclosure;
FIG. 7 illustrates a block diagram of a controller, in accordance with the example embodiment of the present disclosure;
FIGS. 8A-C illustrate retraction of the pressing block by at least one pushing mechanism, in accordance with the example embodiment of the present disclosure; and
FIG. 9 is a flowchart for a method for determining concentration of the target gas, in accordance with the example embodiment of the present disclosure.

### DETAILED DESCRIPTION

Some embodiments will now be described more fully hereinafter with reference to the accompanying drawings, in which some, but not all, embodiments are shown. Indeed, various embodiments may be embodied in many different forms and should not be construed as limited to the embodiments set forth herein; rather, these embodiments are provided so that this disclosure will satisfy applicable legal requirements. As discussed herein, a device, system, and method may be used to measure the concentration of nitric oxide (NO) gas within a sample.

The components illustrated in the figures represent components that may or may not be present in various embodiments of the invention described herein such that embodiments may include fewer or more components than those shown in the figures while not departing from the scope of the invention. Some components may be omitted from one or more figures or shown in dashed line for visibility of the underlying components.

The present disclosure provides various embodiments of measuring and determining concentration of nitric oxide (NO) gas from a target gas taken from a human being. The embodiments disclose about a detecting device, a method, and a system that comprises a sensing chamber and a detecting device that are detachably connected to each other. The sensing chamber is configured to collect and deliver the NO gas to the detecting device that in turn monitors the concentration of the NO gas and displays to a user. Such methods and systems may be used in the medical field such as hospitals, labs, homes etc. for easy, fast, and accurate concentration detection of the NO gas.

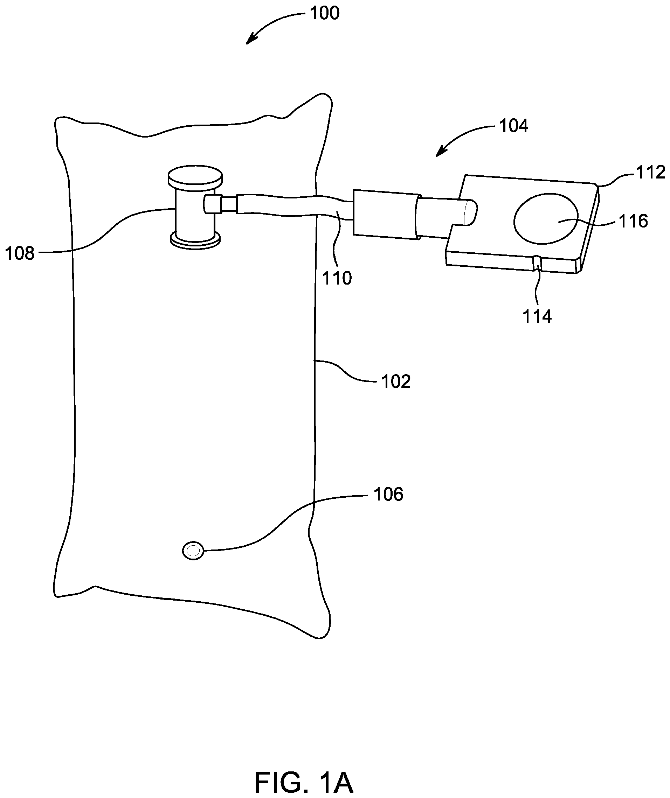

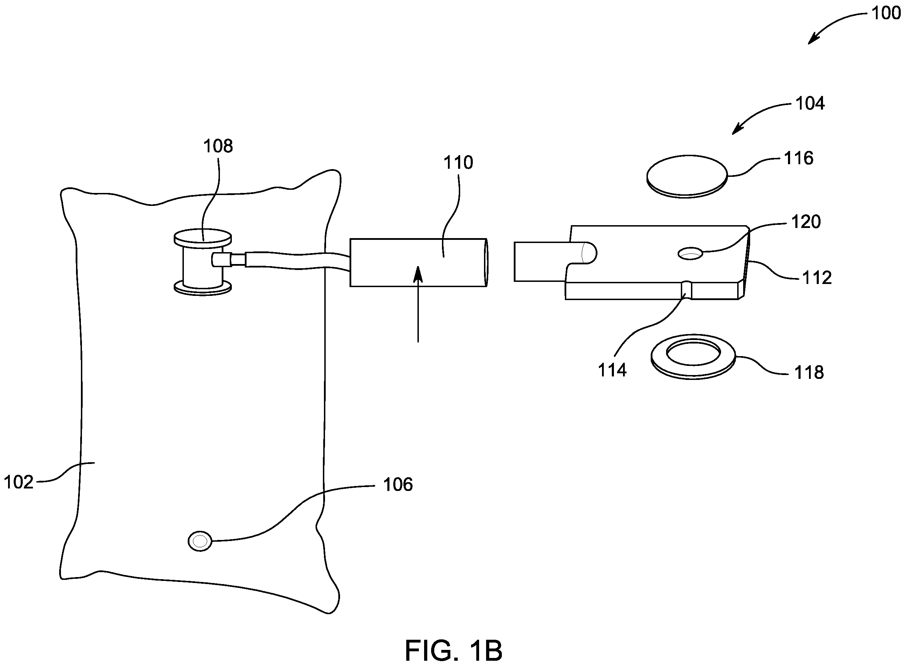

FIG. 1A is a perspective view of a sensing chamber 100 having at least one sampling bag 102 attached to a testing paper module 104, in accordance with an example embodiment of the present disclosure. FIG. 1B is an exploded view of the testing paper module 104 of the sensing chamber 100, in accordance with the example embodiment of the present disclosure. FIG. 1A is described in conjunction with FIG. 1B.

In some embodiments, the sensing chamber 100 may comprise at least one sampling bag 102 and the testing paper module 104. The at least one sampling bag 102 may be configured to receive a target gas from a user. It may be noted that the target gas may include, but is not limited to, nitric oxide (NO) gas, Oxygen (O₂), or Carbon Dioxide (CO₂). In some other embodiments, the target gas may be received from other sources and surroundings, for example clean room, operation room etc. Further, the at least one sampling bag 102 may be fabricated with an elastic material selected from, but is not limited to, rubber, nylon, or plastic. In some embodiments, the at least one sampling bag 102 may be fabricated with at least one nozzle 106 that may provide a passage to fill the target gas inside the at least one sampling bag 102. In some embodiments, the at least one nozzle 106 may be a leak free nozzle. For example, the at least one nozzle 106 may include a single way valve or no-return valve that allows the gas to be received from the user, while not allowing the gas to release or leak from the sampling bag 102.

In some embodiments, the testing paper module 104 may be attached to the at least one sampling bag 102 via a connector 108 and a first tube 110. The connector 108 and the first tube 110 may be configured to facilitate flow of the target gas filled inside the at least one sampling bag 102 to the testing paper module 104. In some embodiments, the connector 108 may be manually attached or detached from the at least one sampling bag 102 via the user. The first tube 110 may be flexible in nature to allow free movement of the testing paper module 104 when attached with the at least one sampling bag 102. Further, the testing paper module 104 may comprise a gas channel 112 fabricated with at least one recess 114, at least one testing paper 116, and a first sealing gasket 118, as shown in FIG. 1B.

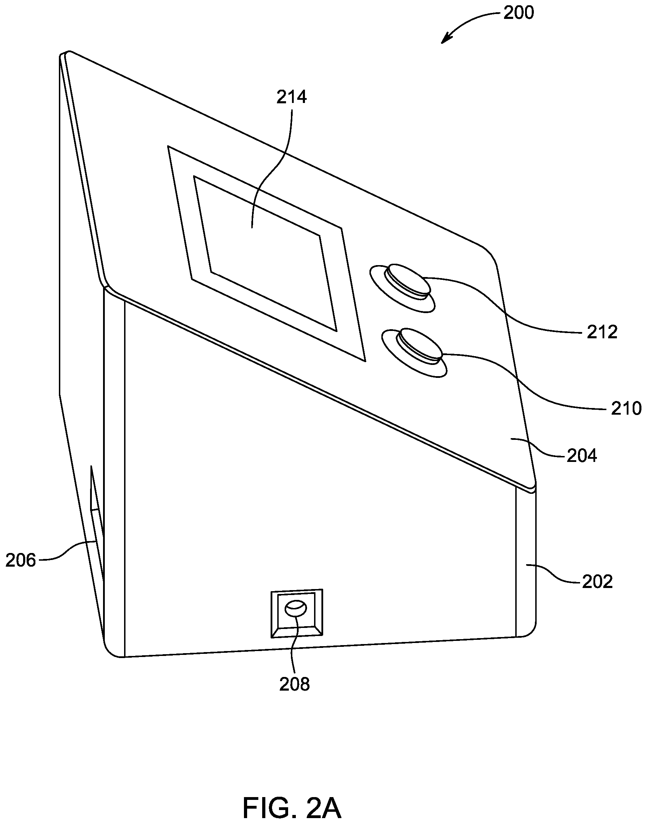

In some embodiments, the gas channel 112 may be connected to the first tube 110 at one end and may be configured to receive the at least on testing paper 116. The gas channel 112 may be configured to channelize the target gas from the at least one sampling bag 102 to the at least one testing paper 116. Further, the gas channel 112 may be fabricated with an opening 120 as shown in FIG. 1B. In some embodiments, the opening 120 may provide a passage for the target gas to flow between the gas channel 112 and the at least one testing paper 116. In some embodiments, the at least one testing paper 116 may be configured to change color based at least on concentration of the target gas received from the gas channel 112. Such change in color of the at least one testing paper 116 based on the concentration of the target gas is described in following embodiments. It may be noted that the detailed working of the at least one recess 114 and the first sealing gasket 118 may be described later in conjunction with FIGS. 2A-2B.

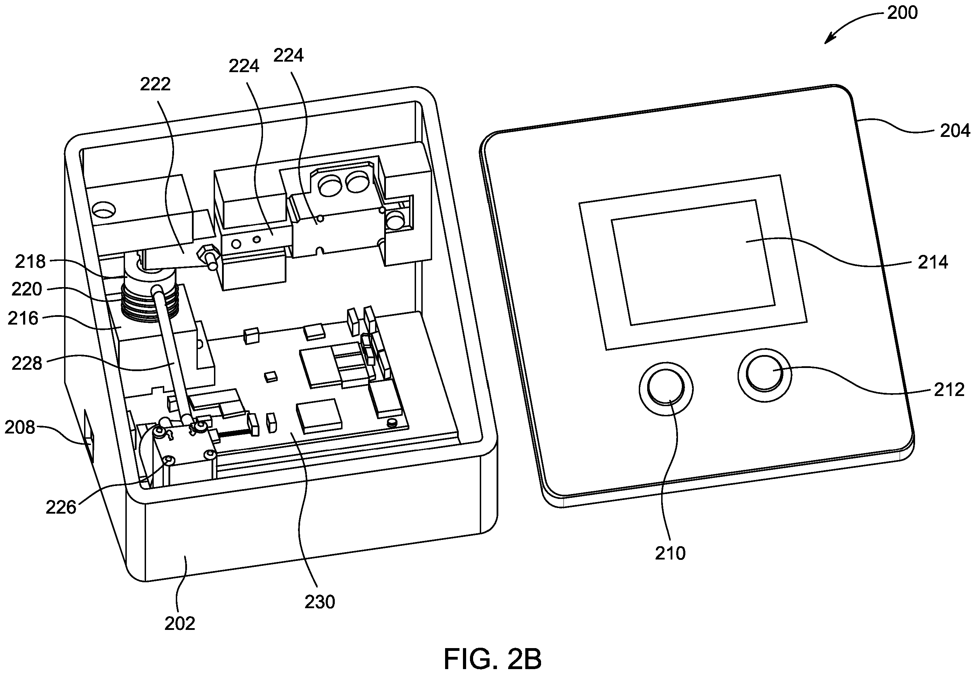

FIG. 2A is a perspective view of a detecting device 200, in accordance with an example embodiment of the present disclosure. FIG. 2B illustrates an internal view of the detecting device 200, in accordance with the example embodiment of the present disclosure. FIGS. 2A-2B are described in conjunction with FIGS. 1A-1B.

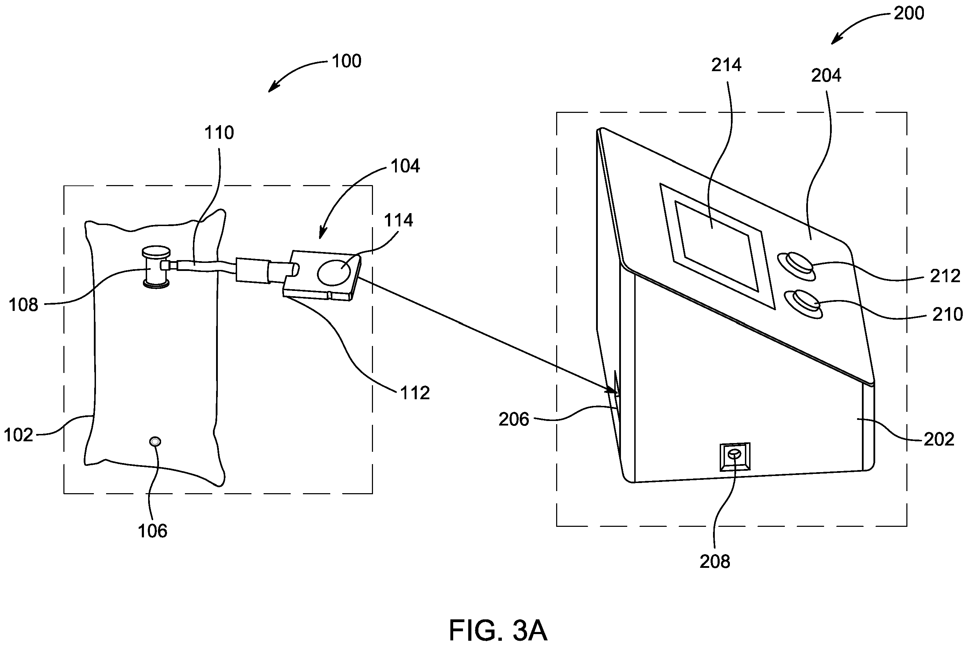

In some embodiments, the detecting device 200 may comprise a housing 202 and a cover 204. The housing 202 may be fabricated with at least one slot 206 and a power input port 208. In some embodiments, the at least one slot 206 may be configured to receive the testing paper module 104 inside the housing 202. Further, the power input port 208 may be configured to plug in a power cable (not shown) into the housing 202 for supplying electrical energy to the detecting device 200. Further, the cover 204 may be fabricated with a power button 210, an on/off button 212, and at least one result screen 214. In some embodiments, the housing 202 may further comprise at least one testing chamber 216, a pressing block 218 coupled to a spring 220, a pushing block 222, at least one pushing mechanism 224, at least one pump 226, a second tube 228, and at least one controller 230. It may be noted that the detailed working of the at least one testing chamber 216, the pressing block 218 coupled to the spring 220, the pushing block 222, at least one pushing mechanism 224, at least one pump 226, the second tube 228, and the at least one controller 230 will be described later in conjunction with FIGS. 3A-5C.

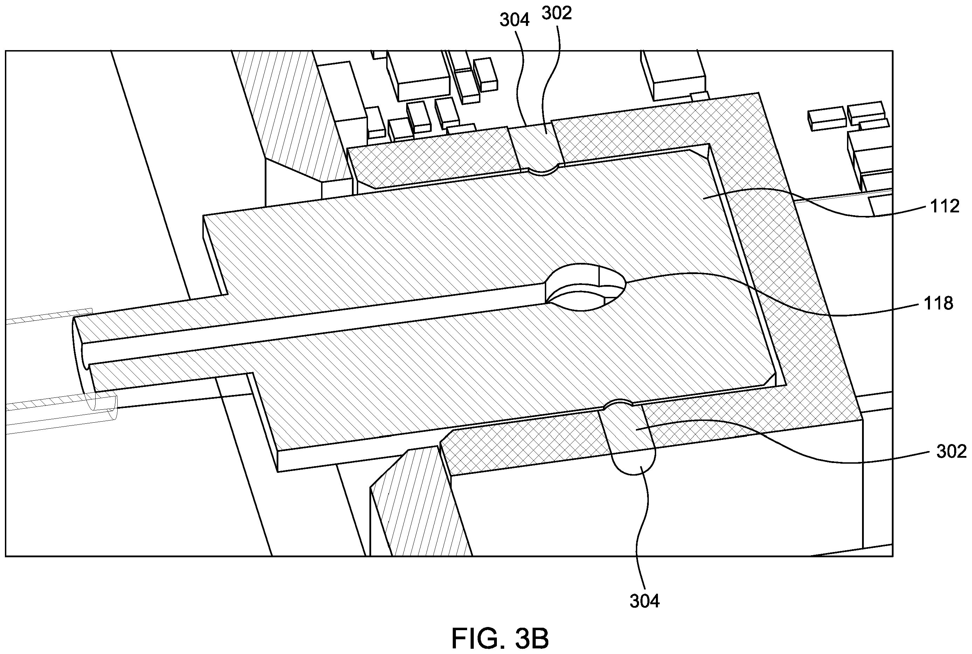

In some embodiments, the testing paper module 104 may be inserted within the detecting device 200 from the at least one slot 206 in order to determine the concentration of the target gas. The at least one slot 206 may be fabricated with at least one locking unit 302, as shown in FIGS. 3A-3B. The at least one locking unit 302 may be configured to fixedly couple the testing paper module 104 within the at least one slot 206. In some embodiments, upon inserting the testing paper module 104, the gas channel 112 may push the at least one locking unit 302. Once the at least one recess 114 as described in the earlier embodiments, reaches parallel to the at least one locking unit 302, the at least one locking unit 302 extends in outward direction as shown by an arrow 304 to interlock with the at least one recess 114 as shown in FIG. 3B. In some embodiments, the at least one locking unit 302 may extend or retract with help of an integrated spring (not shown). Such interlocking of the at least one locking unit 302 couples the testing paper module 104 with the detecting device 200. In some examples, the scope of the disclosure is not limited to the locking unit 302 as is depicted in FIG. 3B. In an example embodiment, the locking unit 302 may be implemented using other snap-fitting techniques, friction locking mechanisms, push to lock mechanism, and/or the like, without departing from the scope of the disclosure.

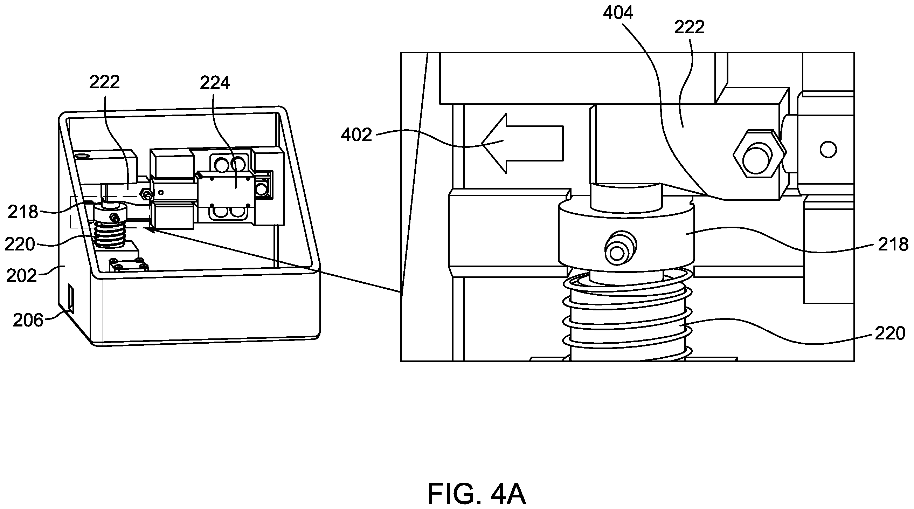

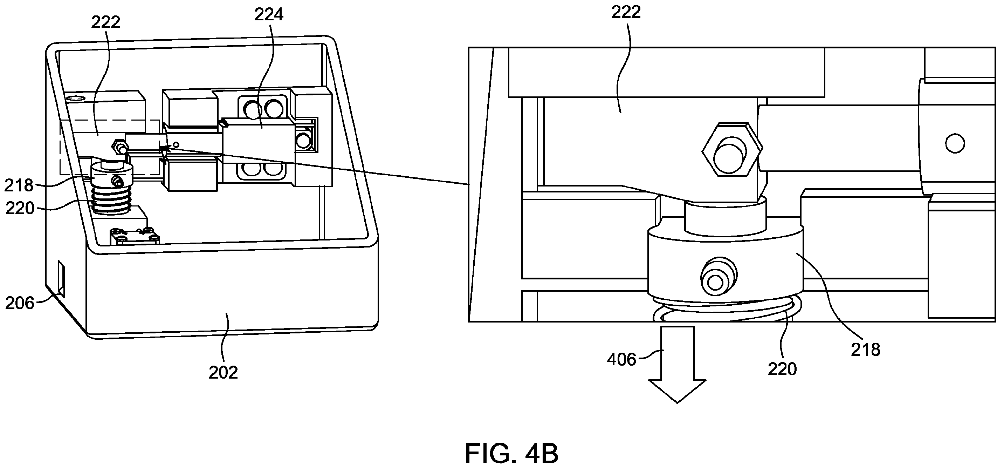

FIG. 4A illustrates horizontal movement of the pushing block 222 with respect to the pressing block 218, in accordance with the example embodiment of the present disclosure. FIG. 4B illustrates vertical movement of the pressing block 218 by the pushing block 222, in accordance with the example embodiment of the present disclosure. FIGS. 4A-4B are described in conjunction with FIGS. 1A-3B.

In some embodiments, the pressing block 218 may be positioned within the housing 202 such that, upon inserting the testing paper module 104 within the at least one slot 206, the pressing block 218 may be vertically positioned above the testing paper module 104 and the at least one testing paper 116. In some embodiments, the pressing block 218 may be configured to channelize flow of the target gas from the testing paper module 104 to the detecting device 200. Further, the pushing block 222 may be mechanically coupled to the at least one pushing mechanism 224. In some embodiments, upon turning "ON" the on/off button 212 by the user, the at least one controller 230 may generate a command signal in order to actuate the at least one pushing mechanism 224. In some other embodiments, the pushing mechanism 224 may be vertically pushed that may further facilitate to push the pressing block 218 directly. The at least one pushing mechanism 224 may be actuated to linearly extend the pushing block 222 as shown by an arrow 402. The linear extension of the pushing block 222 may allow to make a contact with the pressing block 218 with the help of an extended portion 404. In some embodiments, the extended portion 404 upon making a contact with surface of the pressing block 218 may gradually push the pressing block 218 in a downward direction as shown by an arrow 406 in FIG. 4B.

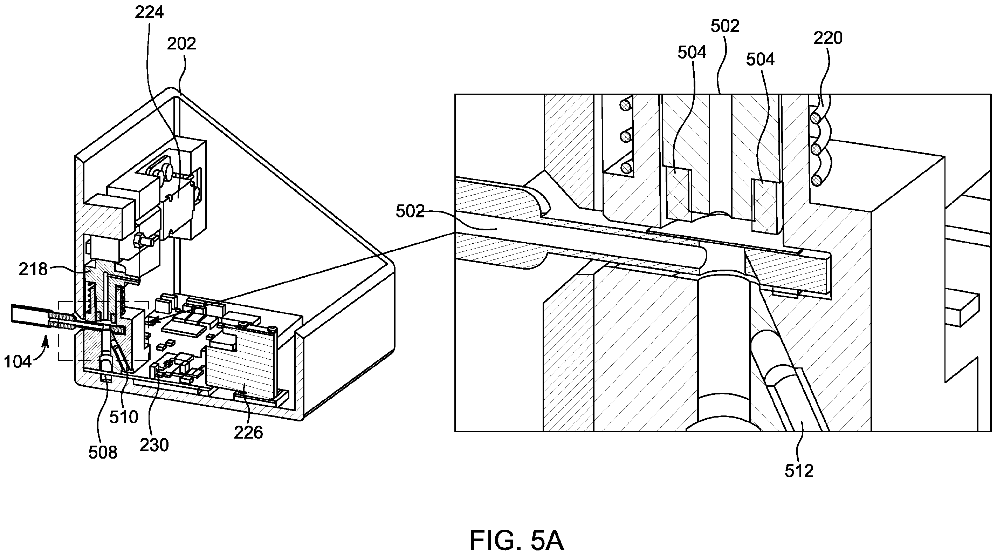

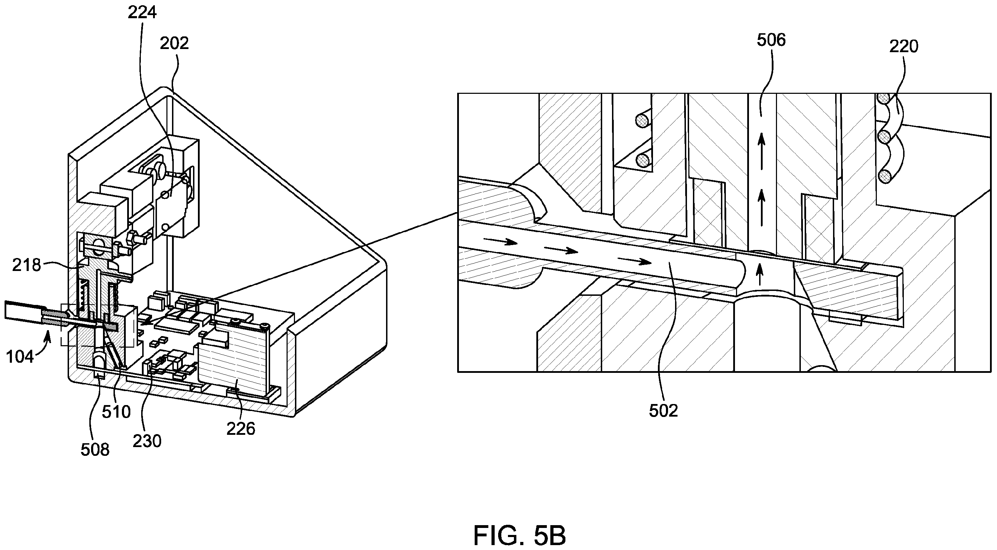

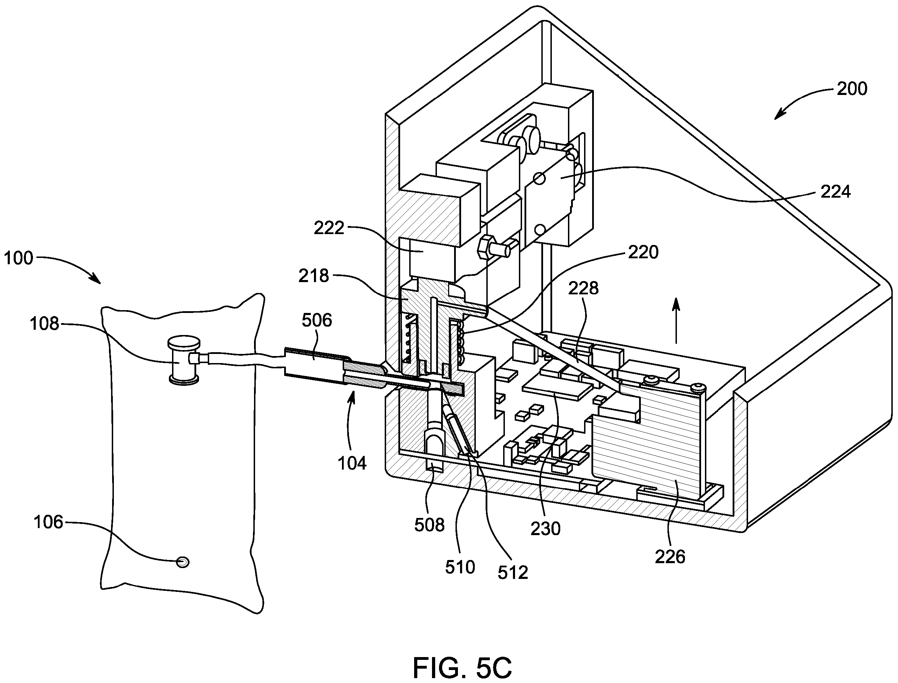

Further, the pressing block 218 once pushed by the pushing block 222, comes in contact and gets placed over the testing paper module 104 as shown in FIG. 5B. In some embodiments, the pressing block 218 may comprise a channel 502. Upon placement of the pressing block 218 over the testing paper module 104, the channel 502 as shown in FIGS. 5A-5B gets placed vertically over the at least one testing paper 116 to allow passing of the target gas from the sensing chamber 100 to the detecting device 200. Further, the pressing block 218 may be fabricated with a second sealing gasket 504. The second sealing gasket 504 may be configured to prevent the target gas from leakage, during the flow of the target gas between the gas channel 112 and the pressing block 218 as shown by an arrow 506 in FIGS. 5B-5C.

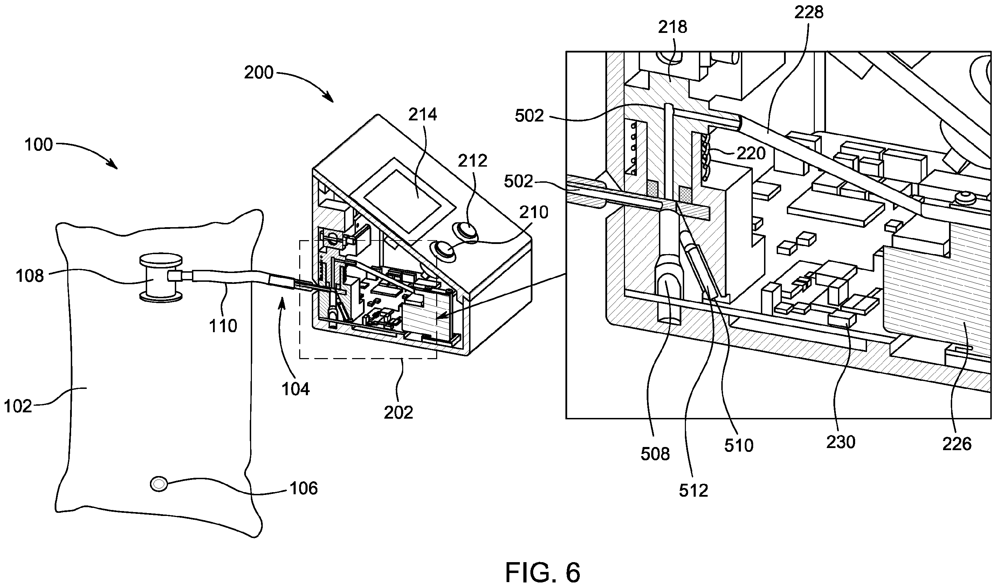

In some embodiments, as described earlier, upon turning "ON" the on/off button 212 by the user, the at least one controller 230 may generate a consecutive command to actuate the at least one pump 226. The at least one pump 226 may be configured to convert an electrical energy into hydraulic energy. Further, the at least one pump 226 may be fluidly linked to the channel 502 via the second tube 228. In some embodiments, the actuation of the at least one pump 226 creates a vacuum through the channel 502. Due to the vacuum inside the channel 502, the target gas starts to flow from the at least one sampling bag 102 through the gas channel 112, the pressing block 218, and into the at least one pump 226 via the second tube 228. The flow of the target gas thereby penetrates the at least one testing paper 116 to allow change in color based on the concentration of the target gas. In some embodiments, the at least one testing paper 116 may be made up of materials such as semiconductor materials, nanomaterials, conductive polymers etc. The materials of the at least one testing paper 116 when comes in contact with the target gas, starts to react due to chemical change. The reaction between the materials of the at least one testing paper 116 and the target gas, results in changing color of the at least one testing paper 116. The change in color of the at least one testing paper 116 may be monitored by at least one detector 508 with the help of at least one illuminating device 510 as shown in FIGS. 5A-5C. It may be noted that the detailed working of the at least one detector 508 and the at least one illuminating device 510 will be described later in conjunction with FIG. 6.

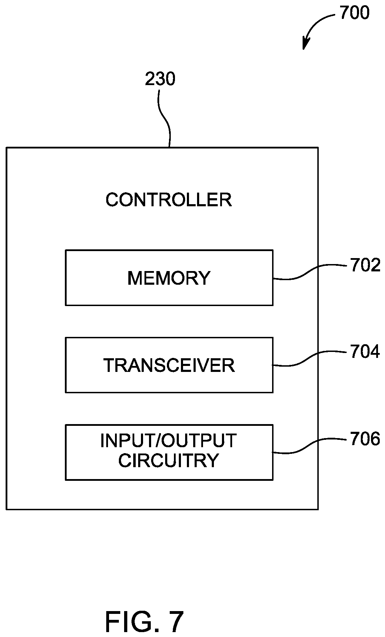

FIG. 6 illustrates monitoring of change in color of the at least one testing paper 116 by the at least one detector 508, in accordance with the example embodiment of the present disclosure. FIG. 7 illustrates a block diagram 700 of a at least one controller 230. FIGS. 6-7 are described in conjunction with FIGS. 1A-5C.

In some embodiments, the housing 202 may further comprise the at least one detector 508 and the at least one illuminating device 510. The at least one illuminating device 510 may correspond to light emitting diode (LED) light. Hereinafter, the at least one illuminating device 510 may be referred to as a LED light. The at least one illuminating device 510 may be configured to illuminate the at least one testing paper 116. The light emitted from the at least one illuminating device 510 may travel through a LED guider 512. The LED guider 512 may facilitate to focus the emitted light from the at least one illuminating device 510 over the at least one testing paper 116. Further, the at least one detector 508 may be configured to monitor the change in color of the at least one testing paper 116. In some embodiments, the at least one detector 508 may emit light and may be reflected by surface of the at least one testing paper 116. The at least one detector 508 may further receive the reflected light from the surface of the at least one testing paper 116.

Further, the reflected light from the at least one testing paper 116 is processed by the at least one controller 230 to determine color values of the reflected light. Further, based on the color values, the at least one controller 230 determines the concentration of the target gas. It may be noted that the at least one detector 508 may preferably a photodetector sensor. Based on the determination, the at least one controller 230 may display the result over the at least one result screen 214. As shown in FIG. 7, the at least one controller 230 includes a memory 702, a transceiver 704, and an input/output circuitry 706.

In some embodiments, the at least one controller 230 (and/or co-processor or any other processing circuitry assisting or otherwise associated with the processor) is/are in communication with the memory 702. In some embodiments, for example, the memory 702 is non-transitory and may include, for example, one or more volatile and/or non-volatile memories. In other words, for example, the memory 702 in some embodiments includes or embodies an electronic storage device (e.g., a computer readable storage medium). In some embodiments, the memory 702 is configured to store information, data, content, applications, instructions, or the like, for enabling the at least one controller 230 to carry out various functions in accordance with example embodiments of the present disclosure.

The at least one controller 230 may be embodied in a number of different ways. For example, in some example embodiments, the at least one controller 230 includes one or more processing devices configured to perform independently. Additionally, or alternatively, in some embodiments, the at least one controller 230 includes one or more processor(s) configured in tandem via a bus to enable independent execution of instructions, pipelining, and/or multithreading. The use of the terms "processor" and "processing circuitry" should be understood to include a single core processor, a multi-core processor, multiple processors internal to the at least one controller 230, and/or one or more remote or "cloud" processor(s) external to the at least one controller 230.

In an example embodiment, the at least one controller 230 is configured to execute instructions stored in the memory 702 or otherwise accessible to the processor. Alternatively, or additionally, the at least one controller 230 in some embodiments is configured to execute hard-coded functionality. As such, whether configured by hardware or software methods, or by a combination thereof, the at least one controller 230 represents an entity (e.g., physically embodied in circuitry) capable of performing operations according to an embodiment of the present disclosure while configured accordingly. Alternatively, or additionally, as another example in some example embodiments, when the at least one controller 230 is embodied as an executor of software instructions, the instructions specifically configure the at least one controller 230 to perform the algorithms embodied in the specific operations described herein when such instructions are executed.

In some embodiments, the at least one controller 230 may be configured to determine the color and/or color shade of the at least one testing paper 116 via image processing algorithms and techniques. The signals received from at least one testing paper 116 by the at least one detector 508 may be received by the at least one controller 230 and processed by the at least one controller 230 to analyze and determine the color and shade. For example, the test paper may turn blue in the presence of the target gas, however, a light shade of below represents a concentration below 1%, a slightly darker shade of blue represents a concentration between 1% and 2% and so on.

In some embodiments, the at least one detector 508 may be configured to detect the color change of the at least one testing paper 116. Initially, the at least one testing paper 116 may be in white color, after the gas concentrates on the at least one testing paper 116, the area of concentration over the at least one testing paper 116 may change to blue color. Further, the at least one detector 508 may thereby identify this color change and may generate different signal. In some embodiment, the at least one controller 230 may compare the signal and determine the concentration of the NO gas.

In some embodiments, the at least one controller 230 includes transceiver 704. The transceiver 704 includes any means such as a device or circuitry embodied in either hardware or a combination of hardware and software that is configured to receive and/or transmit data from/to a network and/or any other device, circuitry, or module in communication with the at least one controller 230. In this regard, the transceiver 704 includes, for example in some embodiments, a network interface for enabling communications with a wired or wireless communications network. Additionally, or alternatively in some embodiments, the transceiver 704 includes one or more network interface card(s), antenna(s), bus(es), switch(es), router(s), modem(s), and supporting hardware, firmware, and/or software, or any other device suitable for enabling communications via one or more communications network(s).

In some embodiments, the at least one controller 230 includes input/output circuitry 706 that provides output to the user and, in some embodiments, to receive an indication of a user input. In some embodiments, the input/output circuitry 706 is in communication with the at least one controller 230 and at least one result screen 214 to provide such functionality. The input/output circuitry 706 may comprise one or more user interface(s) (e.g., user interface) and in some embodiments includes a display that comprises the interface(s) rendered as a web user interface, an application user interface, a user device, a backend system, or the like. The at least one controller 230 and/or input/output circuitry 706 may be configured to control one or more functions of one or more user interface elements through computer program instructions (e.g., software and/or firmware) stored on a memory accessible to the processor (e.g., memory 702, and/or the like). In some embodiments, the input/output circuitry 706 includes or utilizes a user-facing application to provide input/output functionality to a client device and/or other display associated with a user.

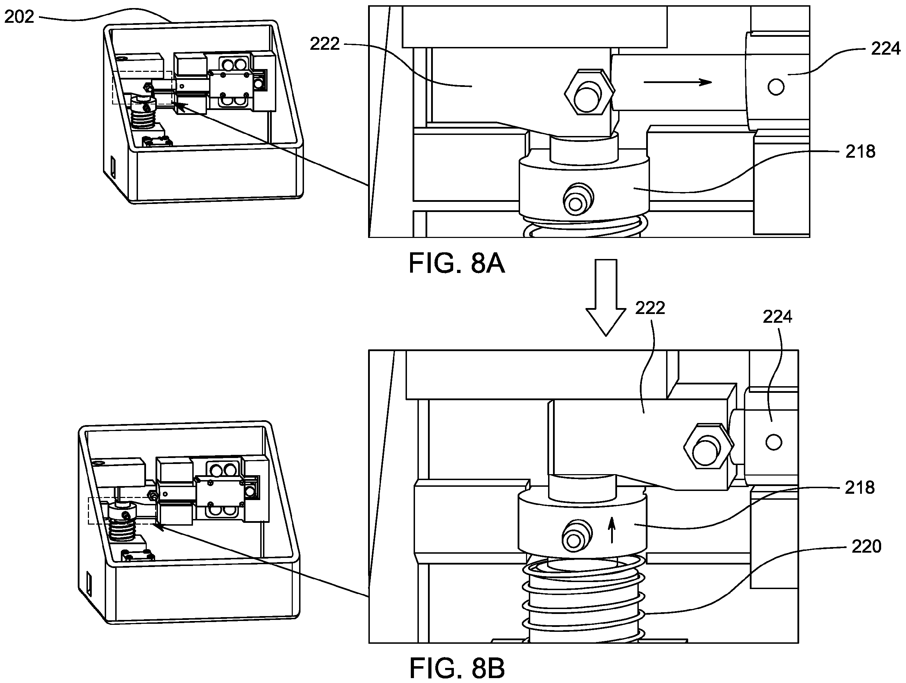

FIGS. 8A-8C illustrate retraction of the pushing block 222 by the at least one pushing mechanism 224, in accordance with the example embodiment of the present disclosure. FIGS. 7A-7C are described in conjunction with FIGS. 1A-6.

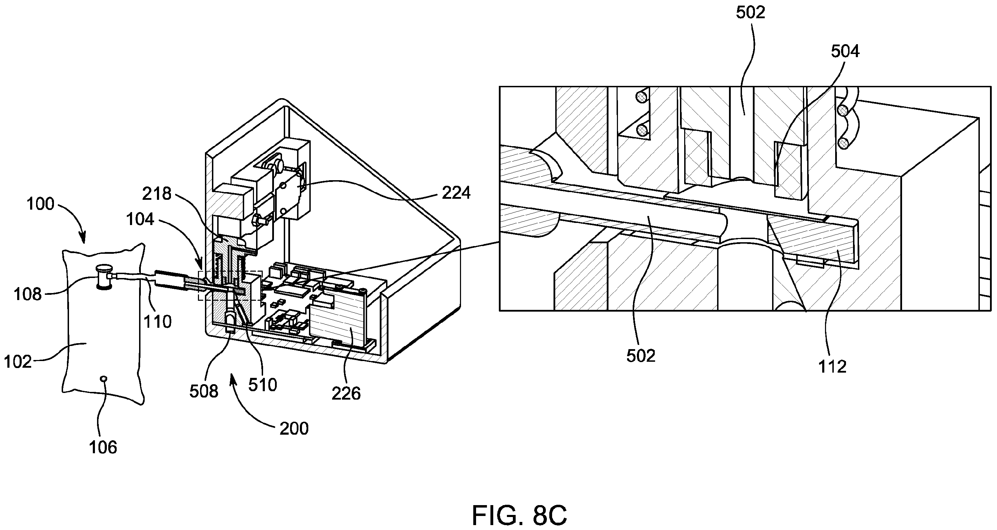

In some embodiments, upon determining and presenting the result over the at least one result screen 214, the user may turn "OFF" the on/off button 212. Upon switching off the on/off button 212, the at least one controller 230 may generate another command to terminate the operation of the at least one pump 226 and further actuate the at least one pushing mechanism 224 to retract the pushing block 222. Such retraction of the pushing block 222 may thereby allow retraction of the pressing block 218 due to the spring 220 as shown in FIG. 8B. In some embodiments, the spring 220 may compress during the vertical downward movement of the pressing block 218 and thereby may store potential energy. Due to the retraction of the pressing block 218, the spring 220 may release the potential energy to push the pressing block 218 in an upward direction. Such upward direction movement may detach the pressing block 218 from the gas channel 112, as shown in FIG. 8C.

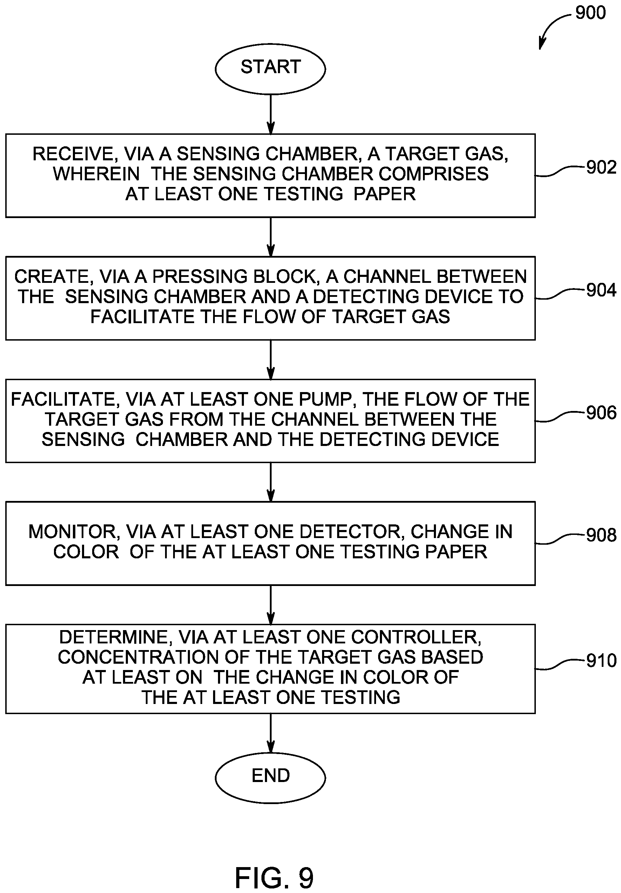

FIG. 9 is a flowchart 900 for a method for determining concentration of the target gas, in accordance with the example embodiment of the present disclosure. FIG. 9 is described in conjunction with FIGS. 1-8C.

At first, the sensing chamber 100 may receive a target gas, at step 902. In some embodiments, the sending chamber 100 may comprise the at least one sampling bag 102 that may be configured to receive target gas. For example, a patient Martin with help of at least one nozzle 106 fills NO gas inside the at least one sampling bag 102. Successively, the pressing block 218 creates the channel 502 between the sensing chamber 100 and the detecting device 200 to facilitate the flow of target gas, at step 904. In some embodiments, upon turning "ON" the on/off button 212 by the user, the at least one controller 230 may generate a command signal in order to actuate the at least one pushing mechanism 224. The at least one pushing mechanism 224 may be actuated to linearly extend the pushing block 222 and thereby push the pressing block 218 to create the channel 502 for facilitating the flow of target gas.

Successively, at least one pump 226 facilitates the flow of the target gas from the channel between the sensing chamber and the detecting device, at step 906. In some embodiments, upon turning "ON" the on/off button 212 by the user, the at least one controller 230 may generate a command to actuate the at least one pump 226. The actuation of the at least one pump 226 may facilitate the target gas to flow from the sensing chamber 100 to the detecting device 200. Successively, at least one detector monitors change in color of the at least one testing paper, at step 908. In some embodiments, the at least one detector 508 may emit light that may be reflected by surface of the at least one testing paper 116 to monitor the change in color. For example, the at least one testing paper 116 turns into blue color. Successively, at least one controller determines concentration of the target gas, based at least on the change in color of the at least one testing paper, at step 910.

In some embodiments, the controller may utilize the image processing algorithms and techniques to identify the test paper, and determine the color and/or color shade of at least one testing paper 116. For example, in some embodiments, at least one testing paper 116 may change colors based on the concentration of the target gas within the target gas. In some embodiments, the color shade of sample gas may represent the concentration of the target gas. For example, the sample gas may turn light blue in the presence of the target gas, however, a light shade of below represents a concentration below 1%, a slightly darker shade of blue represents a concentration between 1% and 2% and so on. The color and/or shading of a correlated concentration may be determined prior to operation and may be input to the controller as a configuration, or operating parameter. In addition, the color and/or shading of a correlated concentration may be learned, for example, through training utilizing known target gases and/or user input.

In some embodiments, the method and the system for measuring and determining concentration of the nitric oxide (NO) gas may be used by doctors and other medical practitioners to regularly monitor the level of NO gas inside a human body. Further, the proposed detecting device, system, and methods are easy to operate and provide rapid and accurate result to a user in a digital form. The size and usability allows patients to use the system without going under the observation of a professional.

Many modifications and other embodiments of the inventions set forth herein will come to mind to one skilled in the art to which these inventions pertain having the benefit of the teachings presented in the foregoing descriptions and the associated drawings. Therefore, it is to be understood that the inventions are not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

## Claims

1. A detecting device (200) comprising:
a housing (202) having at least one testing chamber (216) configured to receive a testing paper module (104) having at least one testing paper (116);
a pressing block (218) inside the housing (202), configured to channelize flow of target gas from the testing paper module (104) to the pressing block (218), wherein the flow of the target gas facilitates to penetrate the target gas through the at least testing paper and change color of the at least one testing paper;
at least one detector (508) configured to monitor the change in color of the at least one testing paper (116); and
at least one controller (230) communicatively coupled to the at least one detector (508) and configured to determine concentration of the target gas based at least on the change in the color of the at least one testing paper (116).

2. The detecting device (200) of claim 1, wherein at least one slot (206) is fabricated over the housing (202) and is configured to insert the testing paper module (104) inside the at least one testing chamber (216).

3. The detecting device (200) of claim 2, wherein the at least one slot (206) further comprises at least one locking unit (302) configured to fixedly couple the testing paper module (104) with the at least one slot (206).

4. The detecting device (200) of claim 1, wherein the housing (202) further comprises:
at least one pump (226) connected to the at least one controller (230) and fluidly coupled to the pressing block (218);
at least one pushing mechanism (224) coupled to the pressing block (218) via a pushing block (222) and electrically coupled to the at least one controller (230),
wherein the at least one pushing mechanism (224) actuates upon receiving a command from the at least one controller (230), that in turn linearly extends the pushing block (222) to vertically actuate the pressing block (218) and position over the at least one testing paper (116), upon activating the detecting device (200); and
the at least one pump (226) actuates to facilitate flow of the target gas to facilitate change in color of the at least one testing paper (116), upon positioning the pressing block (218) over the at least one testing paper (116).

5. The detecting device (200) of claim 1, wherein the housing (202) further comprises:
at least one illuminating device (510) positioned in proximity to the at least one detector (508) and coupled to the at least one controller (230), wherein the at least one illuminating device (510) is configured to illuminate the at least one testing paper (116) to facilitate the at least one detector (508) to monitor the change in color of the at least one testing paper (116).

6. The detecting device (200) of claim 5, wherein the at least one illuminating device (510) comprises at least one of light emitting diode (LED) light (510).

7. The detecting device (200) of claim 1, wherein the housing (202) is enclosed with a cover (204), the cover (204) comprises:
a power button (210) configured to facilitate electrical power to the detecting device (200); and
at least one result screen (214) configured to display the concentration of the target gas to a user.

8. The detecting device (200) of claim 1, wherein the pressing block (218) comprises:
a channel (502) configured to facilitate flow of the target gas within the pressing block (218); and
a sealing gasket (504) configured to seal the channel (502) to prevent leakage of the target gas flowing between the testing paper module (104) and the pressing block (218).

9. The detecting device (200) of claim 1, wherein the pressing block (218) is further coupled to a spring (220) that is configured to retract the pressing block (218) at an initial position upon determining the concentration of the target gas.

10. A method for determining concentration of a target gas, the method comprising:
creating, via a pressing block (218), a channel (502) between a sensing chamber (100) and a detecting device (200) to facilitate flow of the target gas;
facilitating, via at least one pump (226), the flow of the target gas from the channel (502) between the sensing chamber (100) and the detecting device (200);
monitoring, via at least one detector (508), change in color of at least one testing paper (116); and
determining, via at least one controller (230), concentration of the target gas based at least on the change in color of the at least one testing paper (116).

11. The method of claim 10, further comprising,
actuating, via at least one pushing mechanism (224), a pressing block (218) to facilitate translation movement of a pushing block (222); and
pressing, via the pushing block (222), the pressing block (218) to align the pressing block (218) over the at least one testing paper (116) to facilitate flow of the target gas via the at least one testing paper (116).

12. A system for determining concentration of a target gas, the system comprising:
a sensing chamber (100) comprising:
at least one sampling bag (102) configured to receive the target gas; and
a testing paper module (104) having at least one testing paper (116) that is configured to change color based at least on concentration of the target gas;
a detecting device (200) operationally coupled to the sensing chamber (100), comprising:
a housing (202) having at least one testing chamber (216) configured to receive the testing paper module (104); and
at least one controller (230) configured to:
facilitate flow of the target gas through the testing paper module (104) by actuating at least one pump (226), wherein the flow of the target gas facilitates to change color of the at least one testing paper (116); and
determine concentration of the target gas via at least one detector (508), based at least on the change in color of the at least one testing paper (116).

13. The system of claim 12, wherein the testing paper module (104) further comprises a gas channel (112) that is configured to channelize the target gas from the at least one sampling bag (102) to the at least one testing paper (116).

14. The system of claim 13, wherein the gas channel (112) is fluidly coupled to the at least one sampling bag (102) via a first tube (110) to facilitate flow of the target gas from the at least one sampling bag (102) to the at least one testing paper (116).

15. The system of claim 12, wherein the at least one controller (230) is electrically coupled to at least one result screen (214) configured to display the determined concentration of the target gas to a user.
